## Europäisches Patentamt

## European Patent Office

## Office européen des brevets

(19)

(11) Veröffentlichungsnummer: **0 000 518**
**B1**

(12)

# EUROPÄISCHE PATENTSCHRIFT

(45) Veröffentlichungstag der Patentschrift:
**07.01.81**

(21) Anmeldenummer: **78100393.4**

(22) Anmeldetag: **14.07.78**

(51) Int. Cl.³: **C 07 B 19/00,** C 07 C 59/50

(54) **Verfahren zur chemischen Spaltung racemischer Mandelsäure, und D(-)-2-Aminobutanol-(1) Salz der D(-)-Mandelsäure.**

(30) Priorität: **23.07.77 DE 2733425**

(43) Veröffentlichungstag der Anmeldung:
**07.02.79 Patentblatt 79/3**

(45) Bekanntmachung des Hinweises auf die Patenterteilung:
**07.01.81 Patentblatt 81/1**

(84) Benannte Vertragsstaaten:
**BE CH DE FR GB NL**

(56) Entgegenhaltungen:
**HELV.CHIM.ACTA, 26, 929-943 (1943)**
**SAMUEL H. WILEN "Tables of resolving agents and optical resolutions", 1972, University of Notre Dame Press, Notre Dame, Indiana, USA**

(73) Patentinhaber: **RIEDEL-DE HAEN AKTIENGESELLSCHAFT, Wunstorfer Strasse 40, D-3016 Seelze 1 (DE)**

(72) Erfinder: **Dannenberg, Wolfgang, Dr., Düendorfer Weg 20, D-3050 Wunstorf (DE)**
Erfinder: **Schmand, Horst, Dr., Riepener Strasse 17, D-3052 Bad Nenndorf (DE)**

(74) Vertreter: **Meyer-Dulheuer, Karl-Hermann, Dr., HOECHST Aktiengesellschaft Zentrale Patentabteilung Postfach 80 03 20, D-6230 Frankfurt/Main 80 (DE)**

Verfahren zur chemischen Spaltung racemischer Mandelsäure und D(−)-2-Aminobutanol-(1)Salz der D(−)-Mandelsäure

Die Erfindung betrifft ein Verfahren zur chemischen Spaltung racemischer Mandelsäure und Herstellung insbesondere von D(−)-Mandelsäure.

Neben der allgemeinen Verwendbarkeit von optisch aktiven Mandelsäurederivaten zur Racematspaltung von Aminen stellt die D(−)-Mandelsäure ein wichtiges Zwischenprodukt zur Herstellung halbsynthetischer Cephalosporine dar.

Verfahren zur Spaltung von DL-Mandelsäure in die optischen Antipoden sind bekannt. Sie verlaufen über die Bildung diastereomerer Salze mit optisch aktiven Verbindungen, insbesondere mit optisch aktiven Aminen. In den «Tables of Resolving Agents and Optical Resolution», S. H. Wilen, 1972, Notre Dame, Indiana, werden Cinchonin, Cinchonidin, Phenyläthylamin, Ephedrin, Strychnin und Morphin als Spaltungsreagenzien für DL-Mandelsäure aufgeführt.

Bei der Spaltung von DL-Mandelsäure mit Cinchonin unter Verwendung äquimolarer Mengen in Wasser als Lösungsmittel muss die Kristallisation des diastereomeren Salzes durch Animpfen bei einer bestimmten höheren Temperatur eingeleitet werden. Zur Erhöhung der optischen Reinheit sind umfangreiche Umkristallisationen notwendig. Man erhält etwa 80% Ausbeute des Diastereomeren, das nach Spaltung (+)-Mandelsäure ergibt (A. Mackenzie, J. Chem. Soc. 75 [1899] 966).

Bei der Spaltung von DL-Mandelsäure mit (+)-Phenyläthylamin unter Verwendung äquimolarer Mengen in Wasser als Lösungsmittel sind zur Erhöhung der optischen Reinheit auch mehrere Umkristallisationen erforderlich. Es werden etwa 86% Ausbeute an Diastereomeren erhalten, die nach Spaltung des kristallisierten Salzes (−)-Mandelsäure liefern (A.W. Ingersoll et al., J. Amer. Chem. Soc. 55 [1933] 411).

Die hohe Ausbeute lässt sich nur durch eine umfangreiche Aufarbeitung der insgesamt eingesetzten grossen Lösungsmittelmenge erreichen.

Das Verfahren zur Spaltung von DL-Mandelsäure mit (−)-Ephedrin unter Verwendung äquimolarer Mengen in Äthanol als Lösungsmittel lieferte eine Ausbeute von höchstens 85% der Diastereomeren. Nach Spaltung des kristallisierten Salzes erhält man (−)-Mandelsäure. Zur Erhöhung der optischen Reinheit der Mandelsäure sind mehrere Umkristallisationen der Diastereomeren erforderlich. Spätere Nacharbeiten können die Ausbeuteangaben nicht bestätigen. Es werden nur 70% Ausbeute erhalten (R. Roger, J. Chem. Soc. 1935, 1544).

Die Spaltungen mit Morphin, Strychnin und Cinchonidin sind nur unvollständig beschrieben. Strychnin ist ausserdem sehr giftig, so dass eine praktische Verwendung nicht in Betracht kommt.

In den neueren Veröffentlichungen über Racematspaltungen von DL-Mandelsäure tritt neben L-Phenylalanin immer stärker die Spaltung an optisch aktiven Polymeren durch Chromatographie oder Ionenaustauscheffekte in den Vordergrund (G. Blaschke, Chem. Ber. 107, [1974] 237). Das jeweilige Trägermaterial ist dabei mit optisch aktiven Aminen behandelt worden. Die Spaltungen verlaufen jedoch meist unvollständig und haben bislang noch keine technische Verwertung gefunden.

Die in den praktisch nutzbaren Mandelsäure-Racematspaltungen eingesetzten, optisch aktiven Amine sind sehr teuer und haben ausserdem relativ hohe Molekulargewichte, so dass der Einsatz grösserer Mengen notwendig wird.

Es bestand daher die Aufgabe, für die Racematspaltung von DL-Mandelsäure ein billiges Spaltungsmittel mit möglichst niedrigem Molgewicht und hohem Wirkungsgrad bereitzustellen.

Das Prinzip der Racematspaltung durch fraktionierte Kristallisation diastereomerer Salze ist dem Fachmann bereits bekannt. Die fraktioniert kristallisierten Salze sind leicht hydrolysierbar und das gewünschte Enantiomere wird unter Ausnutzung der Säure-Basen-Eigenschaften der Partner von der optisch aktiven Hilfsverbindung getrennt. Eine Voraussage darüber, welche Verbindungen besonders gute Trennungsergebnisse liefern, ist nicht möglich (G.L. Eliel, Stereochemie der Kohlenstoffverbindungen, Weinheim 1966, S. 60ff).

Es wurde nun überraschend gefunden, dass die genannte Aufgabe dadurch gelöst werden kann, dass man DL-Mandelsäure in einem oder einer Mischung mehrerer Lösungsmittel aus der Gruppe Wasser, niedere aliphatische Alkohole und Ketone mit D(−)-2-Aminobutanol-(1) umsetzt, das auskristallisierende diastereomere Salz von der Mutterlauge abtrennt und anschliessend die in Kristallisat und Mutterlauge enthaltenen diastereomeren Salze mit Säuren oder Basen nach an sich bekannten Methoden jeweils in die optisch aktiven Säuren und D(−)-2-Aminobutanol auftrennt.

D(−)-2-Aminobutanol-(1) lässt sich leicht aus dem racemischen 2-Aminobutanol-(1) durch Racematspaltung mit L(+)-Weinsäure gewinnen (DE-PS 1 243 206). Es zeigt eine hohe Racemisierungsbeständigkeit und kann ohne grossen Aufwand durch Destillation gereinigt werden. 2-Aminobutanol-(1) hat ein vergleichsweise niedriges Molekulargewicht und zeigt ausgezeichnete Lösungseigenschaften in den zur Racematspaltung verwendeten Solventien. Nach erfolgreicher Spaltung kann das eingesetzte Amin nach den üblichen Methoden zurückgewonnen und dem Prozess erneut zugeführt werden.

Die optisch aktiven Formen von 2-Aminobutanol-(1) sind bereits als Racematspaltungsreagenzien in Gebrauch. Man musste jedoch auf Grund des Standes der Technik (z.B. Tables of Resolving Agents and Optical Resolution) erwarten, dass nur Basen, die ein grosses Molekül, bevorzugt ein sekundäres oder tertiäres Stickstoffatom, enthalten, für die Racemattrennung der Mandelsäure geeignet sind.

Im vorliegenden Fall ist es überraschend, dass nach der erfindungsgemässen Umsetzung eine Gesamtausbeute von ca. 90% an reinem optisch aktivem Mandelat erhalten werden kann, und zwar weitgehend unabhängig vom verwendeten Lösungsmittel.

Bei Wahl der geeigneten Konzentration ist es möglich, ein diastereomeres Salzpaar zur Ausfällung zu bringen, das bereits ohne Umkristallisation eine hohe, für die Weiterverarbeitung geeignete optische Reinheit aufweist.

Bei Einsatz von D(−)-2-Aminobutanol-(1) kristallisiert aus Wasser, niederen Alkanolen und Ketonen wie z.B. Aceton, das (−).(−)-Salzpaar. Bedingungen für ausreichende Reinheit und Ausbeuten über 90% können durch Variation der Konzentration ermittelt werden. Die Reaktion wird bei Raumtemperatur durchgeführt – das Gemisch erwärmt sich durch exothermen Verlauf der Reaktion – und wird durch anfängliches Rühren zur besseren Durchmischung unterstützt. Animpfen mit entsprechenden Kristallen ist nicht erforderlich, kann aber zur Beschleunigung der Ausfällung empfohlen werden.

Eine Reihe von Versuchen mit verschiedenen Lösungsmitteln und nach Zusammensetzung und Konzentration unterschiedlichen Lösungsmittelgemischen ergab, dass unterschiedliche Konzentrationen Veränderungen in Ausbeute und optischer Reinheit des diastereomeren Salzpaares bewirken. Eine für das jeweilige Lösungsmittel günstigste Konzentration lässt sich durch Experimente eingrenzen. Wasser als Verdünnungsmittel der Alkanole senkt allgemein die Ausbeuten, nimmt aber wenig Einfluss auf die Diastereomerenreinheit der Salze.

Zur Racematspaltung der DL-Mandelsäure kann das optisch aktive Aminobutanol in Anteilen zwischen 0,5 und 1,0 Äquivalenten eingesetzt werden. Bevorzugt werden äquimolare Mengen von Mandelsäure und Aminobutanol verwendet, da auf diese Weise die höchsten Ausbeuten erzielt werden.

Die bei den oben erwähnten Versuchen erhaltenen Produkte wurden zur Reinigung des kristallisierten Salzes mit 5–20 ml Äthanol gewaschen. Durch mehrfache Umkristallisationen wurde $[\alpha]_D^{20} = -80,3°$ als höchste optische Drehung des kristallinen diastereomeren Salzes gemessen. Salze mit spezifischen Drehungen von mindestens −79,0° ergaben nach der sauren Spaltung eine optisch aktive D(−)-Mandelsäure von mindestens 96,5% optischer Reinheit ($[\alpha]_D^{20} = -152,3°$).

Zur Berechnung der optischen Reinheit der Mandelsäure wurden die in der Literatur gefundenen höchsten spezifischen Drehwerte zum Vergleich herangezogen:

$[\alpha]_D^{20,4} = -157,5°$ (Wasser, C = 3,2)
(R. Roger, J. Chem. Soc. 1935, 1544).

$[\alpha]_D^{20} = -157,3°$ (Wasser, C = 1,4)
(Schwab et al., Z. physiol. Chem. 215, [1933] 121).

Für das noch nicht literaturbekannte Salz der D(−)-Mandelsäure mit D(−)-2-Aminobutanol-(1) wurden folgende Kennwerte ermittelt:

Fp = 130°–131° C (Äthanol)
$[\alpha]_D^{20} = -80,3°$ (C = 2, $H_2O$)
$^1$H-NMR ($D_2O$): δ(Na-TMS) 0,90 (t, 3); 1,55 (m, 2); 2,9–3,9 (m, 3); 4,83 (s, 5); 4,98 (s, 1); 745 (m, 5).
Elementaranalyse für $C_{12}H_{19}NO_4$
ber.: C 59,7%; H 7,9%; N 5,8%;
gef.: C 59,8%; H 7,7%; N 5,8%.

Die Erfindung soll durch folgende Beispiele ohne Einschränkung der Allgemeingültigkeit erläutert werden:

Beispiel 1:

In einem 300 ml Erlenmeyer-Kolben werden 60,8 g DL-Mandelsäure (0,4 Mol) in 140 ml Äthanol (95%ig) unter Rühren gelöst. Man fügt 35,6 g D(−)-2-Aminobutanol-(1) (0,4 Mol) hinzu und impft nach guter Durchmischung der Lösung mit wenig D(−)-2-Aminobutanol.D(−)-Mandelat an. Nach erfolgter Kristallisation lässt man über Nacht stehen. Der Niederschlag wird scharf abgesaugt, mit 45 ml Äthanol (95%ig) aufgenommen, kurzfristig zum Sieden erhitzt und nach dem Abkühlen erneut filtriert.

Man wäscht mit 15 ml Äthanol (95%ig) und trocknet bei 50°C im Trockenschrank. Nach dem Trocknen erhält man 43,8 g farbloses D(−)-2-Aminobutanol.D(−)-Mandelat mit Fp. 130–1°C und einer spez. Drehung $[\alpha]_D^{20} = -79,3°$. Die Ausbeute, bezogen auf eingesetzte DL-Mandelsäure beträgt 90,8%. Zur Freisetzung der Mandelsäure werden 43,0 g des D(−)-2-Aminobutanol.D(−)-Mandelats in 150 ml Wasser gelöst und mit 16 ml Salzsäure (37%) auf pH 0,8 eingestellt. Es wird mit 360 ml Diäthyläther (1×120 ml, 4×60 ml) extrahiert. Die organischen Extrakte werden vereinigt, mit Natriumsulfat getrocknet und im Vakuum zur Trockne eingedampft. Nach dem Trocknen erhält man 25,8 g D(−)-Mandelsäure mit Fp. 133°C und einer spez. Drehung von $[\alpha]_D^{20} = -155,2°$ (=98,6% optische Reinheit). Die Ausbeute beträgt 95,0%. Bezogen auf eingesetzte DL-Mandelsäure ergibt sich eine Gesamtausbeute von 84,9%.

Aus dem Filtrat der Racematspaltung mit Aminobutanol wird ohne weitere Isolierung des (−).(+)-Salzes die L(+)-Mandelsäure nach dem gleichen Verfahren mit Salzsäure freigesetzt und mit Diäthyläther extrahiert.

Nach dem Trocknen erhält man 26,5 g L(+)-Mandelsäure mit einer spezifischen Drehung von $[\alpha]_D^{20} = -130,4°$ (=82,8% optische Reinheit). Die Ausbeute beträgt 87,2%, bezogen auf eingesetzte DL-Mandelsäure, wobei die optische Reinheit der L(+)-Mandelsäure zu Lasten der Ausbeute durch fraktionierte Kristallisation aus wenig Diäthyläther noch gesteigert werden kann. Das (−)-2-Aminobutanol-(1) kann nach den üblichen bekannten Methoden, z.B. über Anionenaustauscher, freigesetzt, durch Vakuum-Destillation gereinigt und erneut verwendet werden.

In ähnlicher Weise wurden die folgenden Beispiele ausgeführt, bei denen das Molverhältnis Mandelsäure/Aminobutanol, bzw. die Menge und die Zusammensetzung des Lösungsmittels variiert wurden. Die Ansatzgrösse betrug jeweils 0,1 Mol. Die Fehlerbreite der spez. Drehungen ist mit ±0,5° anzusetzen.

Tabelle

Racematspaltung von DL-Mandelsäure mit (−)-2-Aminobutanol-(1)

| Nr. | Molverhältnis Mandelsäure Aminobutanol | Lösungsmittel und -volumen | Ausbeute % | $[\alpha]_D^{20}$ krist. Salz |
|---|---|---|---|---|
| 2 | 1 | 20 ml Wasser | 58,1 | −79,3° |
| 3 | 1 | 40 ml Methanol | 69,7 | −79,0° |
| 4 | 1 | 30 ml Methanol | 78,1 | −79,3° |
| 5 | 1 | 25 ml Methanol | 92,3 | −62,3° |
| 6 | 1 | 30 ml Methanol/ Wasser (90 : 10) | 70,8 | −78,2° |
| 7 | 1 | 15 ml Methanol/ Wasser (50 : 50) | 72,7 | −77,4° |
| 8 | 1 | 40 ml Äthanol, abs. | 90,45 | −78,2° |
| 9 | 1,32 | 30 ml Äthanol, abs. | 86,3 | −78,7° |
| 10 | 2 | 30 ml Äthanol, abs. | 61,4 | −79,4° |
| 11 | 2 | 40 ml Äthanol 95%ig | 56,4 | −78,5° |
| 12 | 1 | 35 ml Äthanol/ Wasser (95 : 5) | 95,4 | −76,1° |
| 13 | 1 | 40 ml Äthanol/ Wasser (95 : 5) | 91,9 | −77,6° |
| 14 | 1 | 35 ml Äthanol/ Wasser (80 : 20) | 67,2 | −77,8° |
| 15 | 1 | 25 ml Äthanol/ Wasser (50 : 50) | 53,6 | −78,3° |
| 16 | 1 | 120 ml Isopropanol | 97,4 | −74,2° |
| 17 | 1 | 120 ml Isopropanol/ Wasser (90 : 10) | 61,1 | −78,6° |
| 18 | 1 | 70 ml n-Butanol | 95,9 | −75,7° |
| 19 | 1 | 55 ml n-Butanol/ Wasser (80 : 20) | 77,6 | −79,8 |
| 20 | 1 | 150 ml Aceton | 97,1 | −75,6° |

**Patentansprüche**

1. Verfahren zur chemischen Spaltung racemischer Mandelsäure und Herstellung insbesondere von D(−)-Mandelsäure, dadurch gekennzeichnet, dass man DL-Mandelsäure in einem oder einer Mischung mehrerer Lösungsmittel aus der Gruppe Wasser, niedere aliphatische Alkohole und Ketone mit D(−)-2-Aminobutanol-(1) umsetzt, das auskristallisierende diastereomere Salz von der Mutterlauge abtrennt und anschliessend die in Kristallisat und Mutterlauge enthaltenen diastereomeren Salze mit Säuren oder Basen nach an sich bekannten Methoden jeweils in die optisch aktiven Säuren und D(−)-2-Aminobutanol-(1) auftrennt.

2. Salz der D(−)-Mandelsäure mit D(−)-2-Aminobutanol-(1).

**Revendications**

1. Procédé pour dédoubler chimiquement l'acide mandélique racémique et préparer en particulier l'acide D(−) mandélique, procédé caractérisé en ce qu'on fait réagir l'acide DL-mandélique, dans un solvant ou un mélange de plusieurs solvants pris dans l'ensemble constitué par l'eau, des alcools aliphatiques inférieurs et cétones, avec le D(−) amino-2 butanol-1, on sépare de la liqueur-mère le sel diastéréo-isomère qui a cristallisé et on scinde ensuite les sels diastéréo-isomères contenus dans le produit cristallisé et dans la liqueur-mère, au moyen d'acides ou de bases, par des méthodes connues, respectivement en l'un ou l'autre des acides optiquement actifs et en D(−) amino-2 butanol-1.

2. Sel de l'acide D(−) mandélique et du D(−) amino-2 butanol-1.

**Claims**

1. Process for the chemical cleavage of racemic mandelic acid and for the preparation especially of D(−)-mandelic acid, which comprises reacting DL-mandelic acid in one solvent or in a mixture of several solvents of the group water, lower aliphatic alcohols and ketones with D(−)-2-aminobutanol-(1), separating the crystallizing diastereomeric salt from the mother liquor and subsequently splitting according to known methods the diastereomeric salts contained in the crystallization product and the mother liquor with acids or bases to yield each the optically active acids and D(−)-2-aminobutanol-(1).

2. Salt of D(−)-mandelic acid with D(−)-2-aminobutanol-(1).